(19) Europäisches Patentamt
European Patent Office
Office européen des brevets

(11) Publication number: 0 404 076 B1

(12) EUROPEAN PATENT SPECIFICATION

(45) Date of publication of patent specification: 01.03.95

(51) Int. Cl.[6]: C07C 43/12, C07C 41/06

(21) Application number: 90111591.5

(22) Date of filing: 19.06.90

# (54) Process for preparing perhaloethers from perhaloolefins and perhaloethers obtained thereby.

(30) Priority: 20.06.89 IT 2091789

(43) Date of publication of application:
27.12.90 Bulletin 90/52

(45) Publication of the grant of the patent:
01.03.95 Bulletin 95/09

(84) Designated Contracting States:
BE DE FR GB NL

(56) References cited:
EP-A- 0 267 627
EP-A- 0 321 990
US-A- 3 896 167
US-A- 3 985 810
US-A- 4 898 991

(73) Proprietor: AUSIMONT S.p.A.
31, Foro Buonaparte
I-20100 Milano (IT)

(72) Inventor: Marraccini, Antonio, Dr.
3, Via Riviera
I-28040 Dormelletto,
Novara (IT)
Inventor: Pasquale, Antonio
8, C.so Milano
I-28100 Novara (IT)
Inventor: Fiorani, Tiziana
5, Via dei Caccia
I-28100 Novara (IT)
Inventor: Navarrini, Walter, Dr.
46/48 Via A. Moro
I-20010 Boffalora Ticino,
Milan (IT)

(74) Representative: Barz, Peter, Dr. et al
Patentanwälte Dipl.-Ing. G. Dannenberg
Dr. P. Weinhold, Dr. D. Gudel
Dipl.-Ing. S. Schubert, Dr. P.
Barz
Siegfriedstrasse 8
D-80803 München (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 404 076 B1

## Description

The present invention relates to a process for preparing perhaloethers starting from perhaloolefins.

In particular, the present invention relates to a process for preparing perhalomonoethers and perhalopolyethers having defined structure and molecular weight, by reacting at least one perhaloolefin with at least one fluoroxy compound. The invention also relates to new perhaloethers obtainable by the above process.

The compounds obtained may be used in particular in the fields of electric insulating liquids, lubricants and heat transfer media.

Processes for preparing fluorinated polyethers by fluorination of substrates or of hydrogenated polymers and scission of the polymeric chain are known in the art (US-A-4,523,039). This type of process involves long operating times, of the order of a few days, to obtain a complete fluorination.

On the other hand it is known (US-A-3,962,348) to obtain perfluoropolyethers from fluoroolefins and hydrogenated polyols by electrofluorination. Also in this case the process requires very long reaction times and, in addition, the use of HF involves several technological and safety problems etc.

There are also known processes for preparing mixtures of perfluoropolyethers having an undefined molecular weight which is determinable as average molecular weight of the terms contained in the mixtures, on the basis of the ether units present in the chains, by photo-oxidation or polymerization of perfluoroolefins. In this case, too, the processes in question are complicated.

Lastly, it is known to prepare perfluoromonoethers by addition reaction of $CF_3OF$ to olefins, carried out, for example, by using stoichiometric amounts of the reactants at low temperatures and in the presence of ultraviolet light (US-A-4,077,857 and 4,149,016), or in the gas phase at high temperatures (from 20° to 175°C) (Int. J. Chem. Kinet. 1984, 1612, 103-115). Thereby there may be obtained exclusively monoether addition products different from those of the present invention.

It is also known (J. Org. Chem. 1985, 50, 3698-3701) that the use of complex oxidation mixtures consisting of perfluoroacylhypofluorites in combination with mono- and bis-fluoroxy compounds, produced in situ in an aqueous medium and utilized as liquids under pressure as starters for the polymerization of perfluorinated monomers, results in solid polymeric materials having a very high molecular weight.

EP-A-321990, a document relevant under Art. 54(3) EPC, discloses a process for the preparation of chlorotrifluoroethylene telomers wherein chlorotrifluoroethylene is reacted, at -100°C to +40°C, with a fluoroxy compound of formula $R_x$-$CF_2OF$ wherein $R_x$, among others, represents a perhalogenated $C_1$-$C_{10}$ alkyl radical.

EP-A-267627 is directed to a process for the perparation of halogenated polyethers wherein a fluoroxy or chloroxy compound is reacted with a fluorovinylether. In the only example of said document a small percentage of fluorovinylether is first placed in the reactor in the liquid state whereafter the fluoroxy compound diluted with $N_2$ and the remainder of the fluorovinylether are continuously introduced into the reactor, resulting in products containing only one unit derived from the fluorovinylether, i.e., products containing more than one, particularly two, such units are substantially absent.

It has now surprisingly been found that there is a simple and economic method for preparing perhaloethers of defined structure and molecular weight, which is not affected by the drawbacks illustrated in connection with the above prior art.

It was found, in fact, that by reacting a perhalomonoolefin with a fluoroxy compound, both defined in more detail hereinafter, under certain temperature and dilution conditions in an inert gas, it is possible to obtain perhaloether compounds exhibiting a strictly delimited structure and molecular weight.

Said perhaloether compounds are in part new and represent a further surprising feature of the process of the present invention.

Thus, it is an object of the present invention to provide a simple and economic process for preparing perhaloethers (e.g. mono- and polyethers with ether end groups), having defined structure and molecular weight and showing a low degree of oligomerization.

Another object is to provide the perhaloethers also in the form of a directly useful mixture.

Lastly, still another object is to provide "per se" new perhaloethers or mixtures thereof.

These and still further objects, which will become apparent to those skilled in the art from the following description, are achieved, according to the present invention, by means of a process for preparing perhaloethers which process is characterized in that at least one perhalomonoolefin defined below is contacted in the liquid phase which contains the total amount of said perhalomonoolefin(s) and, optionally, an inert organic medium with a stream of inert gas and a stream of at least one fluoroxy compound of formula $R_x$-OF, wherein $R_x$ represents a straight or branched perhaloalkyl radical containing from 1 to 10 carbon atoms, at a temperature of the liquid phase not higher than 20°C.

In the present specification, the fluoroxy compound of formula $R_x$-OF will also be referred to as "starter".

Furthermore, the term "perhalomonoolefin" defines monoolefins in which all of the hydrogen atoms have been substituted by chlorine and fluorine, or only fluorine.

As regards the fluoroxy compound of formula $R_x$-OF, the halogen in the radical $R_x$ may be selected from Cl, F, Br, I and mixtures thereof. Preferably it is selected from F and Cl or only F.

Accordingly, it is possible to use all of the perhalomonoolefins defined below which are compatible with the above process.

The perhalomonoolefins to be employed in the present process are selected from the following groups:

1) one or more perfluoromonoolefins;

2) a fluorochloromonoolefin in combination with a perfluoroalkylvinylether and/or a perfluoromonoolefin;

3) one or more perfluoroalkylvinylethers;

4) a perfluoromonoolefin in combination with a perfluoroalkylvinylether.

Preferably, the fluoroxy compound of formula $R_x$-OF contains from 1 to 3 carbon atoms and, furthermore, the halogen of $R_x$ is selected from chlorine and fluorine or, even better, consists of fluorine only.

As regards the perhalomonoolefins, they preferably contain from 2 to 6 carbon atoms and the halogen is selected, as mentioned hereinbefore, from F and Cl, or fluorine alone.

It is particularly advantageous to use, as perfluoromonoolefin, perfluoropropene and tetrafluoroethylene and, as fluorochloromonoolefin, chlorotrifluoroethylene and 1,2-dichloro-difluoroethylene. Finally, as regards the perfluoroalkylvinylethers, they preferably are of the formula

$CF_2 = CF\text{-}O\text{-}R_f$

wherein $R_f$ represents a straight or branched perfluoroalkyl radical containing from 1 to 10, and preferably from 1 to 3 carbon atoms, such as perfluoromethylvinylether, perfluoroethylvinylether or perfluoropropylvinylether.

The process of the present invention is carried out in a liquid phase of one or more perhalomonoolefins and, optionally, an inert organic medium, employing a (preferably gaseous) stream of the starter or of the starter mixture, an inert gas stream.

Preferably, the inert gas is introduced into the liquid phase in admixture with the gaseous stream of the starter or of its mixtures, in the determined quantitative ratios, and preferably is selected from nitrogen, helium, argon, $CF_4$, $C_2F_6$ and mixtures thereof.

The inert organic solvent medium, when used, preferably comprises linear or cyclic fluorocarbons or chlorofluorocarbons. $CFCl_3$, $CF_2Cl_2$, cyclo-$C_4F_8$, $CF_3$-$CF_2Cl$, $CF_2Cl$-$CFCl_2$ and $CF_2Cl$-$CF_2Cl$ are particularly suitable solvents.

Preferably, the liquid phase is composed of one or more perhalomonoolefins.

The starter of formula $R_x$-OF or mixtures thereof can be employed in combination with minor amounts (ranging from 1 to 30% by mols, preferably from 5 to 20% by mols with respect to $R_x$-OF) of fluorine.

As mentioned before, according to the present invention the reaction temperature is not higher than 20 °C. In other words: the maximum temperature at which the liquid phase is maintained during the reaction shall be such that the component or components of said phase still remain in the liquid state.

The reaction temperature usually ranges from -120 °C to +20 °C and preferably is maintained in the range of from -100 °C to -30 °C. The total pressure generally is maintained around ambient pressure (about 1 atmosphere).

The gaseous volume ratio of the starter or starters to the inert gas can vary over a wide range, for example from 0.01 to 5.

The concentration of the perhalomonoolefin or mixtures thereof in the liquid phase usually ranges from 0.01 to 10 moles/liter of total liquid phase, higher values being allowable up to the molar concentration of the perhalomonoolefin and mixtures thereof in the pure state.

The feeding of the starter or of its mixtures in the gas phase generally is adjusted in such a way as to keep its flow rate in the range of from 0.01 to 5 moles per hour and liter of liquid phase and preferably it ranges from 0.05 to 2 moles per hour and liter of liquid phase.

At the end of the adjusted reaction time, which usually ranges from approximately 2 to 20 hours, the obtained mixture of perhaloethers is separated by distillation from the unreacted perhaloolefin monomer or monomers and from the solvent, if any. By operating in the just described manner, mixtures of perhaloether products are obtained, which products have the appearance of colorless and transparent liquids.

A further separation of the components or of narrower cuts of mixtures, starting from the mixtures of the obtained perhaloether products, can be carried out by fractionated distillation, gas-chromatographic techniques, etc., thereby obtaining products or product cuts having a narrow range, for example, of the boiling points or an analogous boiling point (isomeric mixtures, etc.).

The reaction can be conducted in a completely continuous manner, by continuously withdrawing a liquid phase portion from the reactor, subjecting said portion to distillation and recycling the solvent, if any, and the unreacted monomer or monomers and separating the reaction product.

As mentioned before, the reaction product consists of a mixture of perhalomonoethers and/or perhalopolyethers and optionally of minor amounts of perhaloalkanes, depending on the type of the starting perhalomonoolefin or the mixtures thereof and also depending on the starter(s) used. In most cases said mixtures can be used directly without further separation treatments, etc.

In the following there are described some embodiments of the process of the present invention and the products, or mixtures thereof, obtained thereby, some of said products being novel compounds which are included in the scope of the present invention.

(I) When a perfluoromonoolefin having at least three carbon atoms and, as starter, a fluoroxy compound of formula $R_x$-OF, alone or in combination with elemental fluorine, are used the obtained products have the following formula:

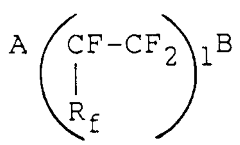

(I)

wherein:

A and B, the same or different from each other, represent $R_xO$; $R_yO$; $R_y$ or F;
$R_f$ represents a straight or branched perfluoroalkyl radical containing from 1 to 10 carbon atoms and preferably from 1 to 3 carbon atoms;
$R_y$ represents a straight or branched perhaloalkyl radical containing at least 1 carbon atom less than $R_x$, and
l is 1 or 2.

Preferably, $R_f$ represents $CF_3$ and $R_y$ is $CF_3$ and/or $C_2F_5$.

The products having formula I, in which l = 2 and A and B are not simultaneously $R_y$ or F, are novel compounds.

In particular, when the starting perhalomonoolefin is only perfluoropropene and the starter is a fluoroxy compound as defined above, alone or in admixture with fluorine, the following products are obtained, in which M represents a monomeric unit derived from perfluoropropene:

$F-(M)_l-F$ (A)
$R_xO-(M)_l-F$ (B)
$R_xO-(M)_l-OR_x$ (C)
$R_y-(M)_l-F$ (D)
$R_y-(M)_l-OR_x$ (E)
$R_yO-(M)_l-F$ (F)
$R_xO-(M)_l-OR_y$ (G)
$R_y-(M)_l-R_y$ (H)

wherein $R_x$ and $R_y$ are defined as above. The products B, C, E, F and G with l = 2 are novel compounds.

In the above case the starting perfluoromonoolefin is perfluoropropene and therefore the monomeric unit M represents a diradical of formula

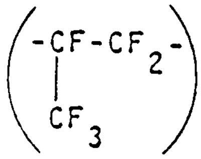

or

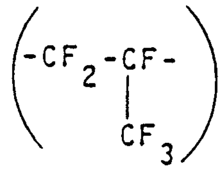

wherefore the products A to H, for example the product (B) with l = 1, exist in two specific isomeric forms having the formulae:

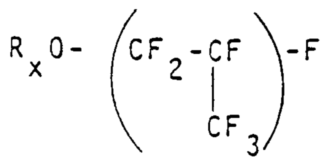

(B1)

and

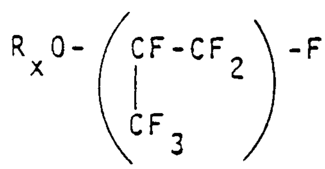

(B2)

Furthermore, for l = 2, all conceivable combinations of isomeric monomeric units are possible wherefore, for example in the case of the product of formula (B), four specific compounds having the following formulae may be present:

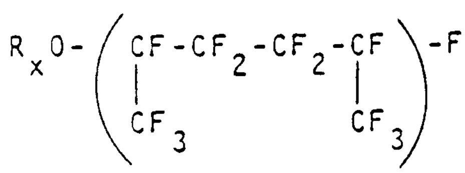

(B3)

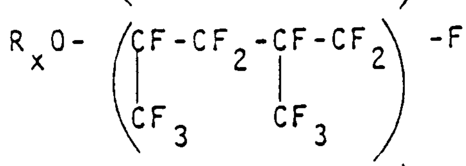

(B4)

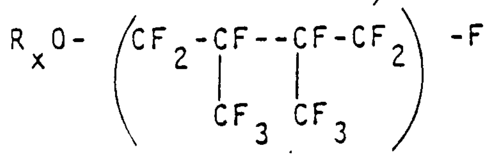

(B5)

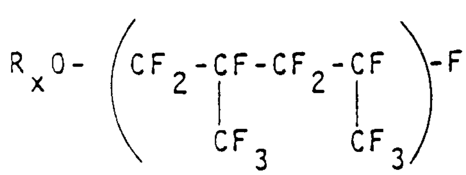

(B6)

The obtained perhaloether mixtures may also contain, generally in small amounts, products different from those indicated above, for example due to the rearrangement of the monomeric unit or units, under

reaction conditions such as to favor local overheating.

For example, when perfluoropropene is employed, alone or in combination, monomeric units of the type

$(-CF_2-CF_2-CF_2-)$ and

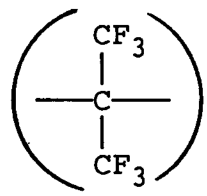

may be present.

Therefore, when perfluoropropene is reacted, as mentioned above, with $CF_3OF$ and elemental $F_2$, the obtained mixture of products A to H is, in particular, composed of:

$CF_3-CF(CF_3)-CF_3$ (1) l = 1
$CF_3-CF_2-CF_2-CF_3$ (2) l = 1
$CF_3-CF_2-CF(CF_3)-CF_3$ (3) l = 1
$CF_3-CF_2-CF_2-CF_2-CF_3$ (4) l = 1
$CF_3O-CF(CF_3)-CF_3$ (5) l = 1
$CF_3O-CF_2-CF_2-CF_3$ (6) l = 1
$CF_3-CF(CF_3)-CF(CF_3)-CF_3$ (7) l = 2
$CF_3-CF(CF_3)-CF_2-CF_2-CF_3$ (8) l = 2
$CF_3(CF_2)_4CF_3$ (9) l = 2
$CF_3-CF(CF_3)-(CF_2)_3CF_3$ (10) l = 2
$CF_3-(CF_2)_2-C(CF_3)_3$ (11) l = 2
$CF_3O-(CF_2)_3-CF(CF_3)_2$ (12) l = 2
$CF_3O-CF_2-CF(CF_3)-CF(CF_3)-CF_3$ (13) l = 2
$CF_3O-CF(CF_3)-CF_2-CF(CF_3)-CF_3$ (14) l = 2
$CF_3O-CF_2CF(CF_3)-(CF_2)_2-CF_3$ (15) l = 2
$CF_3O-CF(CF_3)-(CF_2)_3CF_3$ (16) l = 2
$CF_3O-(CF_2)_6-CF_3$ (17) l = 2
$CF_3O-CF_2-CF(CF_3)-CF(CF_3)-CF_2-OCF_3$ (18) l = 2
$CF_3O-CF_2-CF(CF_3)-CF_2-CF(CF_3)-OCF_3$ (19) l = 2
$CF_3O-CF(CF_3)-CF_2)_2-CF(CF_3)-OCF_3$ (20) l = 2
$CF_3O-CF(CF_3)-(CF_2)_4-OCF_3$ (21) l = 2

The products (11), (12) and (21) are characterized by a rearrangement of the monomeric unit and the products (12) to (21), belonging to the series A to H, are new compounds.

Furthermore, the dimeric products (l = 2) may represent up to 90% and more of the obtained products, of which the mono- and bis-ether products may represent even more than 60%.

When the above reaction is conducted at rather high temperatures, for example at about -30 °C, with a high $CF_3OF$/inert gas volume ratio, for example ≧5, ether products prevailingly consisting of products (12), (13), (17), (18), (20) and (21) are present in the mixture.

Conversely, when the above volume ratio equals about 0.25, the ether products present in the mixture prevailingly consist of products (13) to (16) and (18) to (20).

When perfluoropropene is reacted with $C_2F_5OF$ or a mixture of $C_2F_5OF$ and elemental fluorine, the mixture of the obtained compounds is almost exclusively composed of:

$CF_3-CF(CF_3)-CF(CF_3)-CF_3$ (7) l = 2
$CF_3-CF(CF_3)-CF_2-CF_2-CF_3$ (8) l = 2
$CF_3-(CF_2)_4-CF_3$ (9) l = 2
$CF_3-CF(CF_3)-(CF_2)_3-CF_3$ (10) l = 2
$C_2F_5-O-CF(CF_3)_2$ (1A) l = 1
$C_2F_5O-CF_2-CF_2-CF_3$ (2A) l = 1
$CF_3O-CF_2-CF(CF_3)-(CF_2)_2-CF_3$ (3A) l = 2

$CF_3O\text{-}CF(CF_3)\text{-}(CF_2)_3\text{-}CF_3$ (4A) l = 2
$C_2F_5O\text{-}(CF_2)_3\text{-}CF(CF_3)_2$ (5A) l = 2
$C_2F_5O\text{-}CF(CF_3)\text{-}CF_2\text{-}CF(CF_3)_2$ (6A) l = 2
$C_2F_5O\text{-}CF_2CF(CF_3)\text{-}CF(CF_3)_2$ (7A) l = 2
$C_2F_5O\text{-}CF_2CF(CF_3)\text{-}(CF_2)_2\text{-}CF_3$ (8A) l = 2
$C_2F_5O\text{-}CF(CF_3)\text{-}(CF_2)_3\text{-}CF_3$ (9A) l = 2
$C_2F_5O\text{-}CF_2CF(CF_3)\text{-}CF(CF_3)CF_2CF_3$ (10A) l = 2
$C_2F_5O\text{-}CF_2\text{-}CF(CF_3)\text{-}CF_2\text{-}CF(CF_3)_2$ (11A) l = 2
$C_2F_5O\text{-}CF(CF_3)\text{-}(CF_2)_2\text{-}CF(CF_3)_2$ (12A) l = 2
$C_2F_5O\text{-}CF(CF_3)\text{-}CF_2\text{-}CF(CF_3)\text{-}CF_2\text{-}CF_3$ (13A) l = 2
$C_2F_5O\text{-}CF_2CF(CF_3)\text{-}CF_2\text{-}CF(CF_3)\text{-}OC_2F_5$ (14A) l = 2
$C_2F_5O\text{-}CF_2\text{-}CF(CF_3)\text{-}CF(CF_3)\text{-}CF_2\text{-}OC_2F_5$ (15A) l = 2
$C_2F_5O\text{-}CF(CF_3)\text{-}(CF_2)_2\text{-}CF(CF_3)\text{-}OC_2F_5$ (16A) l = 2

The products (3A) to (16A) are new compounds.

Product (5A) is due to a rearrangement of the monomeric unit.

(II) When a perfluoroalkylvinylether of formula

$R_f\text{-}O\text{-}CF = CF_2$

wherein $R_f$ is defined as above is used as starting monoolefin, the resulting products have the general formula:

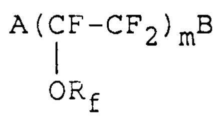

(II)

wherein $R_f$ represents a perfluoroalkyl radical containing from 1 to 10 and preferably from 1 to 3 carbon atoms, as defined before, and m = 1 or 2.

The products having formula II where m = 2 are novel compounds.

When only a perfluoroalkylvinylether is used as starting perfluoromonoolefin and a fluoroxy compound, alone or in admixture with elemental fluorine, is used as starter, the following products, wherein N represents a monomeric unit, are obtained:

$F\text{-}(N)_m\text{-}F$ (A′)
$R_xO\text{-}(N)_m\text{-}F$ (B′)
$R_xO\text{-}(N)_m\text{-}OR_x$ (C′)
$R_y\text{-}(N)_m\text{-}F$ (D′)
$R_y\text{-}(N)_m\text{-}OR_x$ (E')
$R_yO\text{-}(N)_m\text{-}F$ (F')
$R_xO\text{-}(N)_m\text{-}OR_y$ (G')
$R_y\text{-}(N)_m\text{-}R_y$ (H')

wherein $R_x$ and $R_y$ are defined as hereinbefore.

In the formulae of products A' to H', (N) represents a diradical of formulae

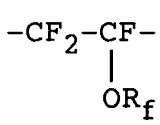

and

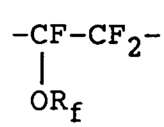

so that, in analogy to what has been discussed above with respect to the reaction with perfluoropropene, two specific products correspond to each formula A' to H' wherein m = 1, and four specific products correspond to each formula A' to H' where m = 2.

When perfluoroalkylvinylethers are employed alone or in combination, monomeric units, resulting from a rearrangement, of the type

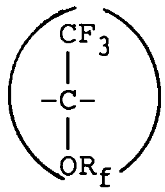 and 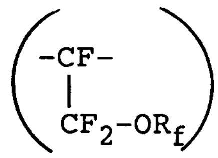

wherein $R_f$ is the same as defined before, may be present.

When perfluoromethylvinylether of formula $CF_3O\text{-}CF = CF_2$ is reacted with $CF_3OF$, alone or in admixture with elemental $F_2$, the mixture of the obtained products is almost exclusively composed of:

$CF_3\text{-}CF(OCF_3)\text{-}CF(OCF_3)\text{-}CF_3$ (1a) m = 2
$CF_3\text{-}CF(OCF_3)\text{-}CF_2\text{-}CF_2\text{-}OCF_3$ (2a) m = 2
$CF_3O\text{-}(CF_2)_4\text{-}OCF_3$ (3a) m = 2
$CF_3O\text{-}CF(OCF_3)\text{-}CF_3$ (4a) m = 1
$CF_3O\text{-}CF_2\text{-}CF_2\text{-}OCF_3$ (5a) m = 1
$CF_3O\text{-}CF(OCF_3)\text{-}CF_2\text{-}CF(OCF_3)\text{-}CF_3$ (6a) m = 2
$CF_3O\text{-}CF(OCF_3)\text{-}(CF_2)_3\text{-}OCF_3$ (7a) m = 2
$CF_3O\text{-}CF_2\text{-}CF(OCF_3)\text{-}CF(OCF_3)\text{-}CF_3$ (8a) m = 2
$CF_3O\text{-}CF_2\text{-}CF(OCF_3)\text{-}(CF_2)_2\text{-}OCF_3$ (9a) m = 2
$CF_3O\text{-}CF(OCF_3)\text{-}CF_2\text{-}CF(OCF_3)\text{-}CF_2\text{-}OCF_3$ (10a) m = 2
$CF_3O\text{-}CF(OCF_3)\text{-}(CF_2)_2\text{-}CF(OCF_3)\text{-}OCF_3$ (11a) m = 2
$CF_3O\text{-}CF_2\text{-}CF(OCF_3)\text{-}CF(OCF_3)\text{-}CF_2\text{-}OCF_3$ (12a) m = 2

These products, with the exception of (5a), are new compounds.

When perfluoromethylvinylether is reacted with $C_2F_5OF$, alone or in admixture with elemental fluorine, the mixture of the obtained products is composed of:

$CF_3\text{-}CF(OCF_3)\text{-}CF_3$ (5) m = 1
$CF_3\text{-}CF_2\text{-}CF_2\text{-}O\text{-}CF_3$ (6) m = 1
$C_2F_5O\text{-}CF(OCF_3)\text{-}CF_3$ (3c) m = 1
$C_2F_5O\text{-}(CF_2)_2OCF_3$ (4c) m = 1
$CF_3\text{-}CF(OCF_3)\text{-}CF(OCF_3)\text{-}CF_3$ (1a) m = 2
$CF_3\text{-}CF(OCF_3)\text{-}(CF_2)_2\text{-}OCF_3$ (2a) m = 2
$CF_3O(CF_2)_4\text{-}OCF_3$ (3a) m = 2
$C_2F_5O\text{-}CF_2\text{-}CF(OCF_3)CF_3$ (5c) m = 2
$C_2F_5O\text{-}CF(OCF_3)\text{-}CF_2\text{-}CF_3$ (6c) m = 2
$CF_3\text{-}CF(OCF_3)\text{-}(CF_2)_3\text{-}OCF_3$ (7c) m = 2
$CF_3\text{-}CF(OCF_3)\text{-}CF_2\text{-}CF(OCF_3)\text{-}CF_3$ (8c) m = 2
$CF_3\text{-}CF_2\text{-}CF(OCF_3)\text{-}CF(OCF_3)\text{-}CF_3$ (9c) m = 2
$CF_3\text{-}CF_2\text{-}CF(OCF_3)\text{-}CF_2\text{-}CF_2\text{-}O\text{-}CF_3$ (10c) m = 2
$C_2F_5O\text{-}CF(OCF_3)\text{-}(CF_2)_3\text{-}OCF_3$ (11c) m = 2
$C_2F_5O\text{-}CF(OCF_3)\text{-}CF_2\text{-}CF(OCF_3)\text{-}CF_3$ (12c) m = 2
$C_2F_5O\text{-}CF_2\text{-}CF(OCF_3)\text{-}CF(OCF_3)\text{-}CF_3$ (13c) m = 2
$C_2F_5O\text{-}CF_2\text{-}CF(OCF_3)\text{-}(CF_2)_2\text{-}OCF_3$ (14c) m = 2
$C_2F_5O\text{-}CF(OCF_3)\text{-}CF_2\text{-}CF(OCF_3)\text{-}CF_2\text{-}O\text{-}C_2F_5$ (15c) m = 2
$C_2F_5O\text{-}CF(OCF_3)\text{-}(CF_2)_2\text{-}CF(OCF_3)\text{-}O\text{-}C_2F_5$ (16c) m = 2
$C_2F_5O\text{-}CF_2\text{-}CF(OCF_3)\text{-}CF(OCF_3)\text{-}CF_2\text{-}O\text{-}C_2F_5$ (17c) m = 2
$C_2F_5\text{-}C(CF_3)(OCF_3)\text{-}CF(CF_3)\text{-}OCF_3$ (18c) m = 2
$CF_3CF(OCF_3)\text{-}CF(OC_2F_5)\text{-}CF_2\text{-}OCF_3$ (19c) m = 2

These products, with the exception of (5), (6) and (4c), are new compounds. Products (18c) and (19c) are characterized by a rearrangement of the monomeric unit.

When perfluoropropylvinylether is reacted with $C_2F_5OF$, alone or in admixture with elemental fluorine, the following new compounds are present in the product mixture:

$C_3F_7\text{-}O\text{-}CF(CF_3)\text{-}O\text{-}C_2F_5$ (1d)
$C_3F_7\text{-}O\text{-}CF_2\text{-}CF_2\text{-}O\text{-}C_2F_5$ (2d)

$C_3F_7$-O-$CF(CF_3)$-$CF(CF_3)$-O-$C_3F_7$ (3d)
$C_3F_7$-O-$CF(CF_3)$-$CF_2$-$CF_2$-O-$C_3F_7$ (4d)
$C_3F_7$-O-$(CF_2)_4$-O-$C_3F_7$ (5d)
$C_2F_5OCF_2CF(OC_3F_7)(CF_2)_2OC_3F_7$ (6d)
$C_2F_5OCF(OC_3F_7)(CF_2)_3OC_3F_7$ (7d)

When perfluoroethylvinylether of formula $C_2F_5$-O-$CF=CF_2$ is reacted with $CF_3OF$, the resulting mixture prevailingly contains the following new products:

$CF_3$-$CF(OC_2F_5)$-$CF(OC_2F_5)$-$CF_3$ (1b)
$CF_3$-$CF(OC_2F_5)$-$(CF_2)_2$-$OC_2F_5$ (2b)
$C_2F_5O$-$(CF_2)_4$-$OC_2F_5$ (3b)

besides compounds (14c), (15c), (16c) and (17c).
(III) when a mixture of a perfluoroalkyl vinylether and a perfluoroolefin is employed as starting monoolefin, products of formula

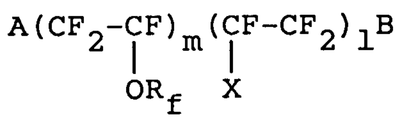 (III)

are obtained, wherein

| | |
|---|---|
| A, B and $R_f$ | are the same as defined hereinbefore, |
| m | is 0, 1, 2; l = 0, 1, 2; m + l = 2; |
| X | represents fluorine or $R_f$, $R_f$ being the same as defined before. |

Preferably, X = F or $CF_3$.

The products with m = l = 1 are new.

In analogy to what has been discussed above, the monomeric units are linked to one another in all possible combinations.

When the above mixture consists of $R_fO$-$CF=CF_2$ and perfluoropropene, the following new products are obtained, in which M represents the perfluoropropene monomeric unit and N the monomeric unit derived from $R_fO$-$CF=CF_2$:

F(M)(N)F (A″)
$R_xO$(M)(N)F (B″)
$R_xO$(M)(N)$OR_x$ (C″)
$R_y$(M)(N)F (D″)
$R_y$(M)(N)$OR_x$ (E″)
$R_yO$(M)(N)F (F″)
$R_xO$(M)(N)$OR_y$ (G″)
$R_y$(M)(N)$R_y$ (H″)

besides the products A to H and A′ to H′.

When $CF_3$-O-$CF=CF_2$ is reacted with perfluoropropene, using $CF_3OF$ alone or in combination with elemental fluorine, the resulting mixture contains, besides the new compounds (1a), (2a), (3a), (13), (15), (16) and (19), the new compounds:

$CF_3O$-$CF_2$-$CF_2$-$CF(CF_3)_2$ (1e)
$CF_3O$-$(CF_2)_4CF_3$ (2e)
$CF_3O$-$CF(CF_3)$-$(CF_2)_2$-$CF_3$ (3e)
$CF_3O$-$CF(CF_3)$-$CF(CF_3)$-$CF_3$ (4e)
$CF_3O$-$CF_2$-$CF(OCF_3)$-$CF_2$-$CF_2$-$CF_3$ (5e)
$CF_3O$-$CF(OCF_3)$-$CF_2$-$CF(CF_3)_2$ (6e)
$CF_3O$-$CF_2$-$CF(OCF_3)$-$CF_2$-$CF(CF_3)$-$OCF_3$ (7e)
$CF_3O$-$CF_2$-$CF(OCF_3)$-$CF(CF_3)$-$CF_2$-$OCF_3$ (8e)

When $CF_3O$-$CF=CF_2$ is reacted with tetrafluoroethylene, using $CF_3OF$ alone or in combination with

elemental fluorine, the resulting mixture contains the new compounds (1a), (2a), (3a), (7a), (9a) and

$CF_3O\text{-}CF(CF_3)\text{-}CF_2\text{-}CF_3$ (1f)
$CF_3O\text{-}(CF_2)_3\text{-}CF_3$ (2f)
$CF_3O\text{-}CF_2\text{-}CF(OCF_3)\text{-}CF_2\text{-}CF_3$ (3f)
$CF_3O\text{-}CF(OCF_3)\text{-}(CF_2)_3CF_3$ (4f)
$CF_3O(CF_2)_4OCF_3$ (5f)
$CF_3O(CF_2)_2\text{-}CF(CF_3)OCF_3$ (6f)

(IV) When a mixture of a perfluoroalkylvinylether and a chlorofluoroolefin selected from CFCl = CFCl and $CF_2$ = CFCl is employed as starting monoolefin, products of formula

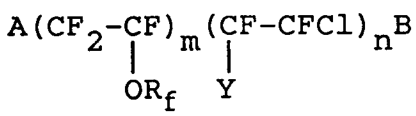

(IV)

are obtained, wherein

A, B and $R_f$ are the same as defined before and m = O, 1, 2; n = 0, 1, 2; m + n = 2;
Y represents F or Cl.

The products with m = n = 1 are new.

In analogy to what has been discussed before, the monomeric units are linked to each other in all possible combinations.

When the above mixture is composed of $R_fO\text{-}CF = CF_2$ and CFCl = CFCl, the following new products are obtained, in which N represents the monomeric unit derived from $R_fO\text{-}CF = CF_2$ and L represents the monomeric unit derived from CFCl = CFCl:

F(N)(L)F ($A'''$)
$R_x$O(N)(L)F ($B'''$)
$R_x$O(N)(L)O$R_x$ ($C'''$)
$R_y$(N)(L)F ($D'''$)
$R_y$(N)(L)O$R_x$ ($E'''$)
$R_y$O(N)(L)F ($F'''$)
$R_x$O(N)(L)O$R_y$ ($G'''$)
$R_y$(N)(L)$R_y$ ($H'''$)

besides the above products A′ to H′.

When $CF_3O\text{-}CF = CF_2$ is reacted with CFCl = CFCl, using $CF_3OF$ alone or in combination with fluorine, the mixture of obtained compounds contains the new products (1a), (2a), (3a) and

$CF_3\text{-}CF(OCF_3)\text{-}CFCl\text{-}CF_2Cl$ (1g)
$CF_3O\text{-}(CF_2)_2\text{-}CFCl\text{-}CF_2Cl$ (2g)
$CF_3O\text{-}CF_2\text{-}CF(OCF_3)\text{-}CFCl\text{-}CF_2Cl$ (3g)
$CF_3O\text{-}CF_2\text{-}CF(OCF_3)\text{-}CFCl\text{-}CFCl\text{-}OCF_3$ (4g)
$CF_3O\text{-}CF(OCF_3)\text{-}CF_2\text{-}(CFCl)_2\text{-}OCF_3$ (5g)

When in the above reaction $CF_2$ = CFCl is used as chlorofluoroolefin, the mixture of obtained compounds contains the new products (1a), (2a), (3a) and

$CF_3O\text{-}CF(CF_3)\text{-}CFCl\text{-}CF_3$ (1h)
$CF_3O\text{-}(CF_2)_2\text{-}CFCl\text{-}CF_3$ (2h)
$CF_3O\text{-}(CF_2)_2\text{-}CF_2\text{-}CF_2Cl$ (3h)
$CF_3O\text{-}CF(CF_3)\text{-}CF_2\text{-}CF_2Cl$ (4h)
$CF_3O\text{-}CF(CF_3)\text{-}CFCl\text{-}CF_2\text{-}OCF_3$ (5h)
$CF_3O(CF_2)_2\text{-}CFCl\text{-}CF_2\text{-}OCF_3$ (6h)

The process of the present invention offers several advantages, which can briefly be summarized as the

possibility of obtaining perhaloethers having defined structure and molecular weight, by means of a simple and flexible process, operating on the concerned parameters, such as the choice of the perhalomonoolefin and the fluoroxy starter compound, in a single reaction step.

The perhalogenated products which contain fluorine and chlorine, obtainable by the process of the present invention, have an important applicative field as electric insulating materials, lubricants and heat transmission media.

The perfluorinated ethers of the invention are compounds which are well known for their exceptional thermal stability, thermooxidative stability and stability towards chemicals as well as their flame-proof properties and are utilizable in very different sectors and under extremely severe operating conditions.

The perfluoropolyethers known in the art generally consist of mixtures of products, from which it is difficult to obtain the individual compounds. In this context GB-A-1,226,566 may be referred to. The perfluoropolyethers of the present invention are generally obtainable as isomeric mixtures of compounds with boiling points in a very narrow temperature range.

The perfluoropolyethers of the invention are particularly useful as fluids for testing in electronics, for example for leak testing, thermal shock testing, hot spot location, dew point determination and the like.

The polyethers containing bromine and/or iodine atoms may be employed as intermediates for the preparation of functionalized derivatives.

The following examples are given for merely illustrative purposes and are not to be regarded as limitative of possible embodiments of the present invention.

EXAMPLE 1

218 g of $C_3F_6$ were condensed in a 500 ml glass reactor equipped with stirrer, thermometer, gas feeding pipes reaching the reactor bottom, and cooler with a liquid at -78°C connected to the atmosphere.

Subsequently, while maintaining external cooling such as to keep the internal temperature at -40°C, a stream of 2.0 Nl/h of $CF_3OF$ and 1 Nl/h of $N_2$ was introduced for 14 hours by bubbling into the liquid phase.

260 g of a crude reaction product were obtained, from which, after having distilled off the unreacted $C_3F_6$ and the volatile by-products, 52 g of a limpid and colorless liquid were obtained, which according to electronic impact gas mass analysis with a 1% SP-1100 column and $^{19}F$-FT-NMR spectrometry, was composed of products 1 to 21. The dimeric (12 to 21) (l = 2) monoether (12-17) and bisether (18 to 21) products, determined by gas-chromatography, amounted to about 55% by weight of the mixture, the weight ratio bisether dimers/monoether dimers being between 1 and 2.

The dimeric monoether products (12-17) have boiling points in the temperature range of -91 ± 2°C at atmospheric pressure.

The dimeric bisether products have boiling points in the temperature range of -121 ± 2°C.

The ether products (12), (17) and (21) were present in trace amounts.

EXAMPLE 2

Example 1 was repeated, using 2.8 Nl/h of $CF_3OF$ diluted with 0.5 Nl/h of $N_2$ bubbled into 186 g of $C_3F_6$ maintained at -40°C.

The obtained products, after removal of $C_3F_6$, were the same as in example 1. The ether terms (12), (13), (17), (18) and (21) represented about 90% of the dimeric ether products obtained. The ether terms (12), (17) and (21) were characterized by a rearrangement of the monomeric unit.

EXAMPLE 3

By using the procedure described in example 1, a stream of 1 Nl/h Of $C_2F_5OF$ and 0.2 Nl/h of $F_2$ diluted with 5 Nl/h Of $N_2$ was bubbled, for 11.5 hours, into 233 g of $C_3F_6$ kept in liquid phase at -48°C.

There were obtained 214 g of a crude reaction mixture which, after $C_3F_6$ removal, was analyzed by means of gas-chromatography, gas-mass and $^{19}F$-NMR. Said mixture was composed of products 1A to 16A. Products 3A to 16A represented about 20% of the mixture. The dimeric monoether products (3A-13A) were present in a weight ratio of about 1:1 with respect to the dimeric bisether products (14A-16A).

EXAMPLE 4

By using the procedure described in example 1, 1 Nl/h of $CF_3OF$ diluted with 3 Nl/h of $N_2$ was bubbled into 230 g of $C_3F_6$ in the liquid state at -60°C and was reacted for 19 hours.

After removal of $C_3F_6$ and the other products which had formed (1 to 11), the dimeric monoethers and diethers (l = 2) were present in a weight ratio of about 1:1.

EXAMPLE 5

By using the procedure described in example 1, a stream of 1 Nl/h of $CF_3OF$ diluted with 5 Nl/h of $N_2$ was bubbled, for 6 hours, into 200 g of $CF_3O\text{-}CF = CF_2$ kept in the liquid state at -60 °C.

190 g of a crude reaction mixture were obtained, from which, by means of distillation, 45 g of ether products (1a) to (12a) were recovered.

EXAMPLE 6

By operating according to the procedure of example 1, after bubbling, for 6 hours, a stream of 1.5 Nl/h of $C_2F_5OF$ diluted with 7.5 Nl/h of $N_2$ into 200 g of $CF_3O\text{-}CF = CF_2$ in the liquid state at -50 °C, 200 g of a crude reaction mixture were obtained, from which, by distillation, 58 g of ether products were recovered; these products, subjected to the above analyses, were composed of products (1a), (2a), (3a), (5), (6) and (3c) to (19c) in the following weight ratios: tetraethers/triethers/diethers = 1/3/6.5.
The products (18c) and (19c) were present in small amounts.

EXAMPLE 7

By operating according to the procedure described in example 1, after bubbling, for 5 hours, of 0.75 Nl/h of $C_2F_5OF$ and of 0.75 Nl/h of $F_2$ diluted with 60 Nl/h of $N_2$ into 112 g of $C_3F_7\text{-}O\text{-}CF=CF_2$ in the liquid state at -50 °C, 127.5 g of a crude reaction mixture were obtained, which mixture was composed of 65% of products (1d) to (7d) and wherein the products (3d) to (5d) represented 20%.

EXAMPLE 8

By operating according to the procedure described in example 1, after bubbling, for 6 hours, a stream of 1 Nl/h of $CF_3OF$ diluted with 3 Nl/h of $N_2$ into 200 g of $C_2F_5\text{-}O\text{-}CF = CF_2$ in the liquid state at -60 °C, and after removal of the unreacted monomer and the volatile by-products, a mixture was obtained, which mixture contained the bisether products (1b) to (3b) and (14c) to (17c).

EXAMPLE 9

By operating according to the procedure described in example 1, after bubbling, for 6 hours, a stream of 2 Nl/h of $CF_3OF$ diluted with 5 Nl/h of $N_2$ into a liquid phase maintained at -60 °C and composed of 115 g of $CF_3O\text{-}CF = CF_2$ and 105 g of $C_3F_6$,and after removal of the unreacted monomers and the volatile by-products, a mixture was obtained, which mixture contained products (1a) to (3a), (13), (15), (16), (19) and (1e) to (8e).

EXAMPLE 10

By operating according to the procedure described in example 1, after bubbling, for 5 hours, 1.5 Nl/h of $CF_3OF$ diluted with 2 Nl/h of $N_2$ into a liquid phase of 55 g of $CF_3O\text{-}CF = CF_2$ and 42 g of CFCl = CFCl maintained at -65 °C, and after removal of the unreacted monomers and of the volatile by-products, a mixture was obtained, which mixture contained products (1a) to (3a) and (1g) to (5g).

EXAMPLE 11

By operating according to the procedure described in example 1, after bubbling, for 5 hours, 1.2 Nl/h of $CF_3OF$ diluted with 3 Nl/h of $N_2$ into a liquid phase of 80 g of $CF_3O\text{-}CF = F_2$ and 57 g of $CF_2$ = CFCl, maintained at -70 °C, and after removal of the unreacted monomers, a mixture was obtained which mixture contained products (1h) to (6h) besides products (1a) to (3a).

EXAMPLE 12

By operating according to the procedure described in example 1 and after bubbling, for 14 hours, 0.5 Nl/h of $CF_3OF$ diluted with 2 Nl/h of $N_2$ into a liquid phase consisting of 50 g of $CF_2Cl$-$CF_2Cl$ and 54.7 g of $C_3F_6$, there were obtained, after removal of the volatile by-products, 8.5 g of a mixture consisting of compounds (5) to (10), (13) to (16) and (18) to (20).

**Claims**

**1.** Process for preparing perhaloethers wherein one or more perhalomonoolefins selected from:
a) a perfluoromonoolefin or a mixture of perfluoromonoolefins,
b) a fluorochloromonoolefin in combination with a perfluoroalkylvinylether and/or a perfluoromonoolefin,
c) a perfluoroalkylvinylether or a mixture of perfluoroalkylvinylethers, and
d) a perfluoromonoolefin in combination with a perfluoroalkylvinylether,
are contacted in the liquid phase containing the total amount of said perhalomonoolefin(s) and, optionally, an inert organic medium and at a temperature of the liquid phase not exceeding 20 °C, with a stream of inert gas and a stream of at least one fluoroxy compound of formula $R_x$-OF, wherein $R_x$ represents a straight or branched perhaloalkyl radical containing from 1 to 10 carbon atoms.

**2.** Process according to claim 1, wherein the perhalomonoolefins contain from 2 to 6 carbon atoms.

**3.** Process according to any one of claims 1 and 2, wherein the group $R_x$ of the fluoroxy compound of formula $R_x$-OF contains from 1 to 3 carbon atoms and the halogen is selected from chlorine, fluorine, bromine, iodine and mixtures thereof, particularly fluorine, alone or in combination with chlorine.

**4.** Process according to any one of the preceding claims, wherein a perfluoromonoolefin or fluorochloromonoolefin selected from perfluoropropene, tetrafluoroethylene, chlorotrifluoroethylene, 1,2-dichlorodifluoroethylene and mixtures thereof is employed.

**5.** Process according to any one of the preceding claims, wherein the perfluoroalkylvinylethers have the formula

$CF_2=CF$-O-$R_f$

wherein $R_f$ represents a straight or branched perfluoroalkyl radical containing 1 to 10 carbon atoms, particularly 1 to 3 carbon atoms.

**6.** Process according to any one of the preceding claims, wherein the inert gas is selected from nitrogen, helium, argon, $CF_4$, $C_2F_6$ and mixtures thereof.

**7.** Process according to any one of the preceding claims, wherein the organic medium comprises at least one linear or cyclic fluorocarbon or chlorofluorocarbon, particularly a halocarbon selected from $CFCl_3$, $CF_2Cl_2$, cyclo-$C_4F_8$, $CF_3CF_2Cl$, $CF_2Cl$-$CFCl_2$, $CF_2Cl$-$CF_2Cl$ and mixtures thereof.

**8.** Process according to any one of the preceding claims, wherein the fluoroxy compounds of formula $R_x$-OF or mixtures thereof are used in combination with elemental fluorine in a molar concentration ranging from 1 to 30% with respect to $R_x$-OF.

**9.** Process according to any one of the preceding claims, wherein the temperature ranges from -120 °C to +20 °C, particularly from -100 °C to -30 °C.

**10.** Process according to any one of the preceding claims, wherein the gaseous volume ratio of the fluoroxy compounds or mixtures thereof to the inert gas ranges from 0.01 to 5.

**11.** Process according to any one of the preceding claims, wherein the concentration of the perhalomonoolefins or mixtures thereof in the liquid phase ranges from 0.01 to 10 moles/liter of total liquid phase.

**12.** Process according to any one of the preceding claims, wherein the fluoroxy compounds or mixtures thereof are fed into the liquid phase at a flow rate ranging from 0.01 to 5 moles, particularly 0.05 to 2 moles, per hour and liter of liquid phase.

**13.** Perhaloethers of formulae I to IV:

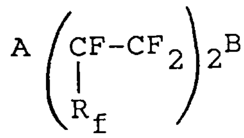

(I)

wherein

- A and B, the same or different from each other, represent $R_xO$, $R_yO$, $R_y$ or F, provided that A and B are not simultaneously $R_y$ or F;
- $R_f$ is as defined in claim 5, and particularly represents $CF_3$;
- $R_x$ is as defined in claims 1, 3 and 4;
- $R_y$ has the meanings given for $R_x$, but contains at least one carbon atom less and particularly represents $CF_3$ and/or $C_2F_5$;

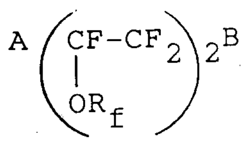

(II)

wherein
A, B and $R_f$ are defined as above;

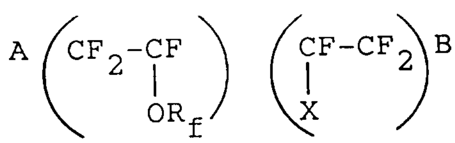

(III)

wherein
A, B and $R_f$ are defined as above and X represents F or $R_f$, particularly $CF_3$;

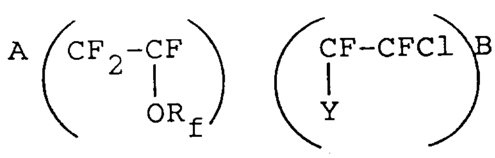

(IV)

wherein
A, B and $R_f$ are as defined above and Y represents F or Cl.

**14.** Perhaloether mixtures, obtainable by the process of claim 1 and consisting essentially of:
(a)

$F-(M)_l-F$ (A)
$R_xO-(M)_l-F$ (B)
$R_xO-(M)_l-OR_x$ (C)

$R_y$-$(M)_l$-F (D)
$R_y$-$(M)_l$-$OR_x$ (E)
$R_y$O-$(M)_l$-F (F)
$R_x$O-$(M)_l$-$OR_y$ (G)

and

$R_y$-$(M)_l$-$R_y$ (H)

wherein

| | |
|---|---|
| $R_x$ and $R_y$ | are defined as in claim 13; |
| l | represents 1 or 2; and |
| M | represents a monomeric unit derived from perfluorpropene; or |

(b)

F-$(N)_m$-F (A')
$R_x$O-$(N)_m$-F (B')
$R_x$O-$(N)_m$-$OR_x$ (C')
$R_y$-$(N)_m$-F (D')
$R_y$-$(N)_m$-$OR_x$ (E')
$R_y$O-$(N)_m$-F (F')
$R_x$O-$(N)_m$-$OR_y$ (G')

and

$R_y$-$(N)_m$-$R_y$ (H')

wherein

| | |
|---|---|
| $R_x$ and $R_y$ | are defined as in claim 13; |
| m | represents 1 or 2; and |
| N | represents a monomeric unit derived from a perfluoroalkylvinylether; |

or
(c)

F(M)(N)F (A'')
$R_x$O(M)(N)F (B'')
$R_x$O(M)(N)$OR_x$ (C'')
$R_y$(M)(N)F (D'')
$R_y$(M)(N)$OR_x$ (E'')
$R_y$O(M)(N)F (F'')
$R_x$O(M)(N)$OR_y$ (G'')
$R_y$(M)(N)$R_y$ (H'')

and the above products A to H and A' to H', wherein

| | |
|---|---|
| $R_x$ and $R_y$ | are defined as in claim 13; |
| M | represents a monomeric unit derived from perfluoropropene; and |
| N | represents a monomeric unit derived from a perfluoroalkylvinylether; |

or
(d)

F(N)(L)F (A''')
$R_x$O(N)(L)F (B''')
$R_x$O(N)(L)$OR_x$ (C''')
$R_y$(N)(L)F (D''')
$R_y$(N)(L)$OR_x$ (E''')
$R_y$O(N)(L)F (F''')
$R_x$O(N)(L)$OR_y$ (G''')
$R_y$(N)(L)$R_y$ (H''')

and the above products A' to H', wherein

$R_x$ and $R_y$ are defined as in claim 13;
N is defined as above; and
L is

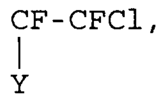

Y being F or Cl.

## Patentansprüche

1. Verfahren zur Herstellung von Perhalogenethern, in welchem ein oder mehrere Perhalogenmonoolefine, die ausgewählt sind aus:
   a) einem Perfluormonoolefin oder einer Mischung von Perfluormonoolefinen,
   b) einem Fluorchlormonoolefin in Kombination mit einem Perfluoralkylvinylether und/oder einem Perfluormonoolefin,
   c) einem Perfluoralkylvinylether oder einer Mischung von Perfluoralkylvinylethern und
   d) einem Perfluormonoolefin in Kombination mit einem Perfluoralkylvinylether,

   in der flüssigen Phase, die die gesamte Menge an Perhalomonoolefin(en) und gegebenenfalls ein inertes organisches Medium enthält, bei einer Temperatur der flüssigen Phase nicht über 20 °C mit einem Inertgasstrom und einem Strom mindestens einer Fluoroxyverbindung der Formel $R_x$-OF, worin $R_x$ einen geraden oder verzweigten Perhalogenalkylrest, der 1 bis 10 Kohlenstoffatome enthält, darstellt, in Kontakt gebracht werden.

2. Verfahren nach Anspruch 1, in welchem die Perhalogenmonoolefine 2 bis 6 Kohlenstoffatome enthalten.

3. Verfahren nach irgendeinem der Ansprüche 1 und 2, in welchem die Gruppe $R_x$ der Fluoroxyverbindung der Formel $R_x$-OF 1 bis 3 Kohlenstoffatome enthält und das Halogen aus Chlor, Fluor, Brom, Iod und Mischungen davon, insbesondere Fluor, allein oder in Kombination mit Chlor, ausgewählt ist.

4. Verfahren nach irgendeinem der vorangehenden Ansprüche, in welchem ein Perfluormonoolefin oder Fluorchlormonoolefin, das aus Perfluorpropen, Tetrafluorethylen, Chlortrifluorethylen, 1,2-Dichlordifluorethylen und Mischungen davon ausgewählt ist, eingesetzt wird.

5. Verfahren nach irgendeinem der vorangehenden Ansprüche, in welchem die Perfluoralkylvinylether die Formel

   $CF_2$ = CF-O-$R_f$

   aufweisen, worin $R_f$ für einen geraden oder verzweigten Perfluoralkylrest, der 1 bis 10 Kohlenstoffatome, insbesondere 1 bis 3 Kohlenstoffatome, enthält, steht.

6. Verfahren nach irgendeinem der vorangehenden Ansprüche, in welchem das Inertgas aus Stickstoff, Helium, Argon, $CF_4$, $C_2F_6$ und Mischungen davon ausgewählt wird.

7. Verfahren nach irgendeinem der vorangehenden Ansprüche, in welchem das organische Medium mindestens einen linearen oder cyclischen Fluorkohlenstoff oder Chlorfluorkohlenstoff, insbesondere einen Halogenkohlenstoff, der ausgewählt ist aus $CFCl_3$, $CF_2Cl_2$, cyclo-$C_4F_8$, $CF_3CF_2Cl$, $CF_2Cl$-$CFCl_2$, $CF_2Cl$-$CF_2Cl$ und Mischungen davon, umfaßt.

8. Verfahren nach irgendeinem der vorangehenden Ansprüche, in welchem die Fluoroxyverbindungen der Formel $R_x$-OF oder Mischungen davon in Kombination mit elementarem Fluor in einer molaren Konzentration im Bereich von 1 bis 30% bezüglich $R_x$-OF eingesetzt werden.

**9.** Verfahren nach irgendeinem der vorangehenden Ansprüche, in welchem die Temperatur im Bereich von -120 °C bis +20 °C, insbesondere -100 °C bis -30 °C, liegt.

**10.** Verfahren nach irgendeinem der vorangehenden Ansprüche, in welchem das Gas-Volumenverhältnis der Fluoroxyverbindungen oder Mischungen davon zum Inertgas im Bereich von 0,01 bis 5 liegt.

**11.** Verfahren nach irgendeinem der vorangehenden Ansprüche, in welchem die Konzentration der Perhalogenmonoolefine oder Mischungen davon in der flüssigen Phase im Bereich von 0,01 bis 10 Mol/Liter der gesamten flüssigen Phase liegt.

**12.** Verfahren nach irgendeinem der vorangehenden Ansprüche, in welchem die Fluoroxyverbindungen oder Mischungen davon mit einer Fließgeschwindigkeit im Bereich von 0,01 bis 5 Mol, insbesondere 0,05 bis 2 Mol, pro Stunde und Liter der flüssigen Phase in die flüssige Phase eingeführt werden.

**13.** Perhalogenether der Formeln I bis IV:

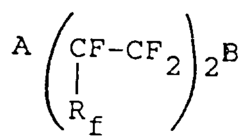

(I)

worin

| | |
|---|---|
| A und B, | gleich oder verschieden voneinander, $R_xO$, $R_yO$, $R_y$ oder F darstellen, mit der Maßgabe, daß A und B nicht gleichzeitig $R_y$ oder F sein können; |
| $R_f$ | wie in Anspruch 5 definiert ist und insbesondere $CF_3$ darstellt; |
| $R_x$ | wie in den Ansprüchen 1, 3 und 4 definiert ist; |
| $R_y$ | die für $R_x$ angegebenen Bedeutungen aufweist, aber mindestens ein Kohlenstoffatom weniger enthält und insbesondere für $CF_3$ und/oder $C_2F_5$ steht; |

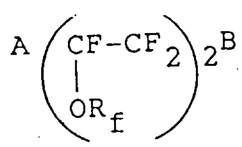

(II)

worin
A, B und $R_f$ wie oben definiert sind;

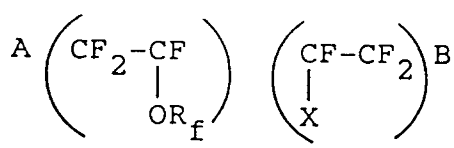

(III)

worin A, B und $R_f$ wie oben definiert sind und X für F oder $R_f$, insbesondere $CF_3$, steht;

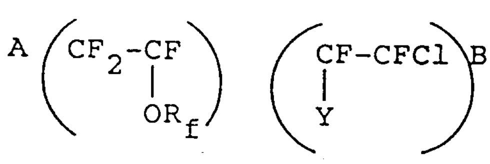

(IV)

worin A, B und $R_f$ wie oben definiert sind und Y für F oder Cl steht.

**14.** Perhalogenether-Mischungen, erhältlich durch das Verfahren von Anspruch 1 und im wesentlichen bestehend aus:

(a)

$F-(M)_l-F$ (A)
$R_xO-(M)_l-F$ (B)
$R_xO-(M)_l-OR_x$ (C)
$R_y-(M)_l-F$ (D)
$R_y-(M)_l-OR_x$ (E)
$R_yO-(M)_l-F$ (F)
$R_xO-(M)_l-OR_y$ (G)

und

$R_y-(M)_l-R_y$ (H)

worin

| | |
|---|---|
| $R_x$ und $R_y$ | wie in Anspruch 13 definiert sind; |
| l | 1 oder 2 darstellt; und |
| M | für eine von Perfluorpropen abgeleitete Monomer-Einheit steht; oder |

(b)

$F-(N)_m-F$ (A')
$R_xO-(N)_m-F$ (B')
$R_xO-(N)_m-OR_x$ (C')
$R_y-(N)_m-F$ (D')
$R_y-(N)_m-OR_x$ (E')
$R_yO-(N)_m-F$ (F')
$R_xO-(N)_m-OR_y$ (G')

und

$R_y-(N)_m-R_y$ (H')

worin

| | |
|---|---|
| $R_x$ und $R_y$ | wie in Anspruch 13 definiert sind; |
| m | 1 oder 2 darstellt; und |
| N | für eine von einem Perfluoralkylvinylether abgeleitete Monomer-Einheit steht; |

oder

(c)

$F(M)(N)F$ (A'')
$R_xO(M)(N)F$ (B'')
$R_xO(M)(N)OR_x$ (C'')
$R_y(M)(N)F$ (D'')
$R_y(M)(N)OR_x$ (E'')
$R_yO(M)(N)F$ (F'')
$R_xO(M)(N)OR_y$ (G'')
$R_y(M)(N)R_y$ (H'')

und den obigen Produkten A bis H und A' bis H', worin

| | |
|---|---|
| $R_x$ und $R_y$ | wie in Anspruch 13 definiert sind; |
| M | für eine von Perfluorpropen abgeleitete Monomer-Einheit steht; und |
| N | für eine von einem Perfluoralkylvinylether abgeleitete Monomer-Einheit steht; |

oder

(d)

F(N)(L)F (A''')
$R_xO(N)(L)F$ (B''')
$R_xO(N)(L)OR_x$ (C''')
$R_y(N)(L)F$ (D''')
$R_y(N)(L)OR_x$ (E''')
$R_yO(N)(L)F$ (F''')
$R_xO(N)(L)OR_y$ (G''')
$R_y(N)(L)R_y$ (H''')

und den obigen Produkten A' bis H', worin

$R_x$ und $R_y$ wie in Anspruch 13 definiert sind;
N wie oben definiert ist; und
L für

CF-CFCl
|
Y

steht, wobei Y F oder Cl darstellt.

## Revendications

1. Procédé de préparation de perhaloéthers dans lequel on met une ou plusieurs perhalomonooléfines choisies parmi:
   a) une perfluoromonooléfine ou un mélange de perfluoromonooléfines,
   b) une fluorochloromonooléfine en combinaison avec un perfluoroalkylvinyléther et/ou une perfluoromonooléfine,
   c) un perfluoroalkylvinyléther ou un mélange de perfluoroalkylvinyléthers et
   d) une perlfuoromonooléfine en association avec un perfluoroalkylvinyléther,

   phase liquide contenant la quantité totale de la ou desdites perhalomonooléfines et éventuellement un milieu organique inerte et à une température de la phase liquide ne dépassant pas 20 °C, au contact d'un courant de gaz inerte et d'un courant d'au moins un dérivé fluoroxy de formule $R_x$-OF, dans laquelle $R_x$ représente un radical perhaloalkyle à chaîne droite ou ramifiée contenant de 1 à 10 atomes de carbone.

2. Procédé selon la revendication 1, dans lequel les perhalomonooléfines contiennent de 2 à 6 atomes de carbone;

3. Procédé selon l'une quelconque des revendications 1 et 2, dans lequel le groupe $R_x$ du dérivé fluoroxy de formule $R_x$-OF contient de 1 à 3 atomes de carbone et l'halogène est choisi parmi chlore, fluor, brome, iode et leurs mélanges, en particulier fluor seul ou en combinaison avec le chlore.

4. Procédé selon l'une quelconque des revendications précédentes dans lequel une perfluoromonooléfine ou fluorochloromonooléfine choisie parmi perfluoropropène, tétrafluoroéthylène, chlorotrifluoroéthylène, 1,2-dichloro-difluoroéthylène et leurs mélanges est employé.

5. Procédé selon l'une quelconque des revendications précédentes dans lequel les perfluoroalkylvinyléthers présentent la formule

   $CF_2 = CF\text{-}O\text{-}R_f$

   dans laquelle $R_f$ représente un radical perfluoroalkyle à chaîne droite ou ramifiée contenant de 1 à 10 atomes de carbone, en particulier de 1 à 3 atomes de carbone.

6. Procédé selon l'une quelconque des revendications précédentes dans lequel le gaz inerte est choisi parmi azote, hélium, argon, $CF_4$, $C_2F_6$ et leurs mélanges.

7. Procédé selon l'une quelconque des revendications précédentes dans lequel le milieu organique comprend au moins un fluorocarbone ou chlorofluoroacarbone linéaire ou cyclique, en particulier un halocarboné choisi parmi $CFCl_3$, $CF_2Cl_2$, cyclo-$C_4F_8$, $CF_3CF_2Cl$, $CF_2Cl$-$CFCl_2$, $CF_2Cl$-$CF_2Cl$ et leurs mélanges.

8. Procédé selon l'une quelconque des revendications précédentes, dans lesquelles les dérivés fluoroxy de formule $R_x$-OF ou leurs mélanges sont utilisés en combinaison avec du fluor élémentaire en concentration molaire de 1 à 30% par rapport à $R_x$-OF.

9. Procédé selon l'une quelconque des revendications précédents, dans lequel les températures sont comprises entre -120 et +20°C, en particulier -100 et -30°C.

10. Procédé selon l'une quelconque des revendications précédentes dans lequel le rapport du volume gazeux des dérivés fluoroxy ou de leurs mélanges au gaz inerte est compris entre 0,01 et 5.

11. Procédé selon l'une quelconque des revendications précédentes dans lequel la concentration des perhalomonooléfines ou de leurs mélanges dans la phase liquide est comprise entre 0,01 et 10 moles/litre de phase liquide total.

12. Procédé selon l'une quelconque des revendications précédentes, dans lequel les dérivés fluoroxy ou leurs mélanges sont introduits dans la phase liquide à un débit compris entre 0,01 et 5 moles par heure et par litre, en particulier entre 0,05 et 2 moles par heure et par litre de phase liquide.

13. Perhaloéthers de formule I à IV:

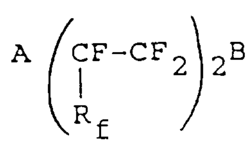

(I)

dans laquelle

- A et B, identiques ou différents l'un de l'autre, représentent $R_xO$, $R_yO$, $R_y$ ou F, pourvu que A et B ne représentent pas simultanément $R_y$ ou F;
- $R_f$ est tel que défini dans la revendication 5 et en particulier représente $CF_3$;
- $R_x$ est tel que défini dans les revendications 1, 3 et 4.
- $R_y$ a les significations données pour $R_x$ mais contient au moins un atome de carbone de moins et en particulier représente $CF_3$ et/ou $C_2F_5$;

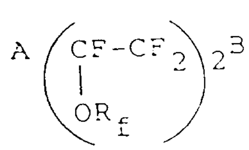

(II)

dans laquelle

A, B et $R_f$ sont tels que définis ci-dessus;

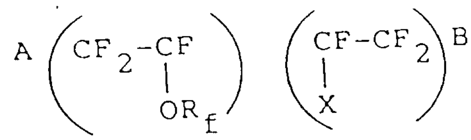

(III)

dans laquelle A, B et $R_f$ sont tels que définis ci-dessus et X représente F ou $R_f$, en particulier $CF_3$;

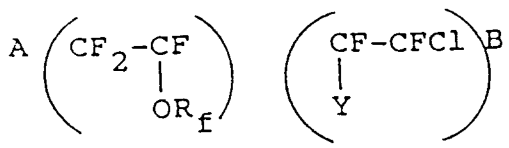

(IV)

dans lequel A, B et $R_f$ sont tels que définis ci-dessus et Y représente F ou Cl.

**14.** Mélanges de perhaloéthers pouvant être obtenus par le procédé selon la revendication 1 et constitués essentiellement de:

(a)

$F\text{-}(M)_l\text{-}F$ (A)
$R_xO\text{-}(M)_l\text{-}F$ (B)
$R_xO\text{-}(M)_1\text{-}OR_x$ (C)
$R_y\text{-}(M)_l\text{-}F$ (D)
$R_y\text{-}(M)_l\text{-}OR_x$ (E)
$R_yO\text{-}(M)_l\text{-}F$ (F)
$R_xO\text{-}(M)_l\text{-}OR_y$ (G)

et

$R_y\text{-}(M)_l\text{-}R_y$ (H)

dans lesquelles $R_x$ et $R_y$ sont tels que définis dans la revendication 13; l représente 1 ou 2; et M représente un motif monomère dérivé du perfluoropropène; ou

(b)

$F\text{-}(N)_m\text{-}F$ (A')
$R_xO\text{-}(N)_m\text{-}F$ (B')
$R_xO\text{-}(N)_m\text{-}OR_x$ (C')
$R_y\text{-}(N)_m\text{-}F$ (D')
$R_y\text{-}(N)_mOR_x$ (E')
$R_yO\text{-}(N)_m\text{-}F$ (F')
$R_xO\text{-}(N)_m\text{-}OR_y$ (G')

et

$R_y\text{-}(N)_m\text{-}R_y$ (H')

dans lesquelles $R_x$ et $R_y$ sont tels que définis dans la revendication 13; m représente 1 ou 2; et N représente un motif monomère dérivé d'un perfluoroalkylvinyléther; ou

(c)

F(M)(N)F (A'')
$R_x$O(M)(N)F (B'')
$R_x$O(M)(N)O$R_x$ (C'')
$R_y$(M)(N)F (D'')
$R_y$(M)(N)O$R_x$ (E'')
$R_y$O(M)(N)F (F'')
$R_x$O(M)(N)O$R_y$ (G'')
$R_y$(M)(N)$R_y$ (H'')

et les produits ci-dessus A à H et A' à H' dans lesquels $R_x$ et $R_y$ sont tels que définis dans la revendication 13;

M représente un motif monomère dérivé du perfluoropropène; et

N représente un motif monomère dérivé d'un perfluoroalkylvinyléther;

ou

(d)

F(N)(L)F (A''')
$R_x$O(N)(L)F (B''')
$R_x$O(N)(L)O$R_x$ (C''')
$R_y$(N)(L)F (D''')
$R_y$(N)(L)O$R_x$ (E''')
$R_y$O(N)(L)F (F''')
$R_x$O(N)(L)O$R_y$ (G''')
$R_y$(N)(L)$R_y$ (H''')

et les produits ci-dessus A' à H', dans lesquels $R_x$ et $R_y$ sont tels que définis dans la revendication 13;

N est tel que défini ci-dessus; et

L représente

$$\begin{array}{l} CF-CFCl, \\ | \\ Y \end{array}$$

Y étant F ou Cl.